Europäisches Patentamt

European Patent Office

Office européen des brevets

(11) Publication number: **0 380 229**
**A1**

# (12) EUROPEAN PATENT APPLICATION

(21) Application number: **90300453.9**

(22) Date of filing: **17.01.90**

(51) Int. Cl.5: **A61B 17/41**

(30) Priority: **27.01.89 GB 8901835**

(43) Date of publication of application:
**01.08.90 Bulletin 90/31**

(84) Designated Contracting States:
**AT BE CH DE DK ES FR GR IT LI LU NL SE**

(71) Applicant: **Lewis, Lionel Maurice**
**8 Chantry Close**
**Kenton, Harrow, Middlesex(GB)**

(72) Inventor: **Lewis, Lionel Maurice**
**8 Chantry Close**
**Kenton, Harrow, Middlesex(GB)**

(74) Representative: **Jones, Graham H.**
**Graham Jones & Company 77 Beaconsfield**
**Road Blackheath**
**London SE3 7LG(GB)**

(54) **Depilation needle.**

(57) A depilation needle comprising an elongate conductive body (1) the surface of which is covered with insulating material (2), the body (1) having at one end a conductive tip (4) suitable for depilation purposes and at the other end a conductive surface portion (5) which communicates electrically with the tip (4) via the body (1), the needle being such that during depilation the conductive tip (4) and the insulating material (2) adjacent the conductive tip (4) are able to contact a person's skin whereby the insulating material (2) prevents the person's skin receiving an electrical depilation current except from the conductive tip (4), and the insulating material (2) comprising a non-toxic polyamide/polyimide resin.

FIG 1

## DEPILATION NEEDLE

This invention relates to depilation needles for use with electrical depilatory apparatus.

Electrical depilatory apparatus comprising a depilatory needle which is used to pass an electrical current through a hair follicle whereby the hair follicle is destroyed is well known. Such apparatus normally comprises a stainless steel needle which is used to contact the hair follicle and through which a current is passed to kill the hair follicle, thereby to prevent further growth of hair therefrom. One of the problems associated with electrical depilation is that the skin surrounding the hair follicle may be marked, albeit temporarily due to current flowing through surface tissue. This marking of the skin usually manifests itself as scabs and red blotches and it is clearly undesirable. Over a period of years, scarring may appear as the pores shrink as a result of the treatment.

In United Kingdom patent No. 2136296 there is described and claimed a depilation needle which obviates or reduces the above mentioned problem. The depilation needle in the patent is covered with a special type of insulating material.

I have now discovered a particularly advantageous insulating material that may be used on the depilation needle and that is easy to apply to the needle, is non-toxic and is generally satisfactory in use.

Accordingly, this invention provides a depilation needle comprising an elongate conductive body the surface of which is covered with insulating material, the body having at one end a conductive tip suitable for depilation purposes and at the other end a conductive surface portion which communicates electrically with the tip via the body, the needle being such that during depilation the conductive tip and the insulating material adjacent the conductive tip are able to contact a person's skin whereby the insulating material prevents the person's skin receiving an electrical depilation current except from the conductive tip, and the insulating material comprising a non-toxic polyamide/polyimide resin.

The resin is relatively inert and it is non-metallic.

The insulating material may include a lubricant material and/or a pigment. In this connection, the insulating material preferably includes polytetrafluoroethylene as a lubricant material, and carbon black as a pigment.

The resin may be that sold under the Trade Mark Xylan. Preferably, the Xylan is Xylan 8110 or Xylan 8114. if a black coating is required, then Xylan 8110/870 Black may be used. Xylan polyamide/polyimide resins are available from the Whitford Corporation, of West Chester, Pennsylvania, United States of America.

The insulating material will usually be in the form of a lacquer or varnish. The lacquer or varnish will usually dry to a hard inert coating on the depilation needle. The insulating material may be cured quite quickly at elevated temperatures. The insulating material may also be cured at lower temperatures, for example at ambient temperature, but then a longer curing time will usually be required.

The insulating material does not involve any intricate or complicated techniques in its application to the depilation needles. The insulating material may thus be applied simply by printing, spraying or dipping the depilation needles into the insulating material. Electrostatic deposition may also be employed. The insulating material does not appear to have any harmful effects on the skin such for example as skin irritation. Also, the insulating material does not appear to be susceptible to harbouring micro organisms that might tend to generate infections in a person's skin.

The depilation needle of the present invention can thus be used such that a current is passed along the needle so as to bypass surface layers of skin and to pass into the body at a region adjacent the hair follicle. Thus it is simple to apply to a follicle a suitable depilation current without damaging the surface tissues underlying it.

The needle may be bent or cranked. The needle may be made of stainless steel.

The tip should ideally be about one millimetre in length.

The tip may comprise a cylindrical extension of the body having a reduced diameter in order to permit insertion between a hair and its pore.

Embodiments of the invention will now be described solely by way of example and with reference to the accompanying drawings in which:

Figure 1 is a somewhat schematic sectional view of depilation apparatus including a needle; and

Figure 2 is a somewhat schematic sectional view of depilation apparatus which includes a needle of alternative design.

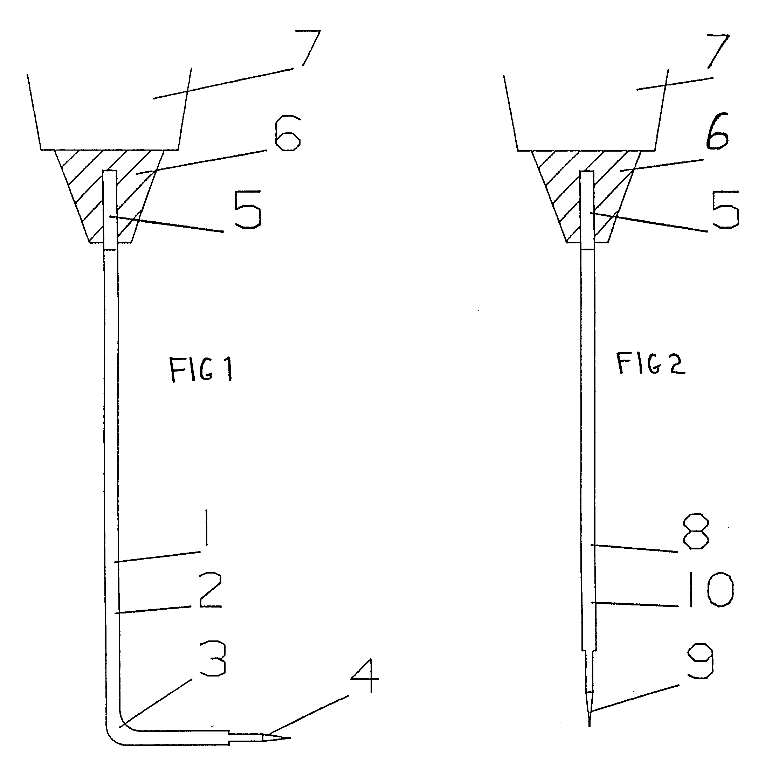

Referring now to Figure 1, a depilation needle comprises a first surgical stainless steel body portion 1 which is generally circular in cross section and which is coated with a thin insulative material coating 2. The coating 2 comprises a polyamide/polyimide resin mixed with polytetrafluoroethylene as a lubricant/release agent and carbon black as a pigment. The coating 2 forms a hard inert coating.

The body portion 1 is bent or crinked at a region 3 and extends to define an insulated surgical stainless steel depilation tip 4 at one end. The end of the body portion 1 remote from the tip 4 is also uninsulated and comprises a shank portion 5 which is received into a plated brass chuck 6 through which depilation currents are fed to the needle. The metal chuck 6 is supported in a body 7 which is fabricated from an insulating material such as a plastics material.

The coating 2 can be provided on the body portion 1 by various methods including dipping the body portion 1 into the insulating material, or painting or spraying the insulating material on to the body portion 1. Usually, when the body portion 1 has been provided with the insulating material, the insulating material will be dried at ambient or elevated temperatures to form the coating 2.

Referring now to Figure 2 wherein parts corresponding to those shown in Figure 1 bear the same numerical designations, a needle 8 is provided which is similar to the needle shown in Figure 1 but which is straight and which does not therefore include the cranked region 3. As can be seen most clearly in Figure 2, the needle 8 includes an end part which includes an uninsulated tip 9 which is generally finer than the main body portion 10 of the needle 8 and which can therefore be inserted into the skin so that the whole of the conductive tip 9 lies beneath the surface of the skin next to the bulb of a hair follicle to be destroyed.

It will be appreciated that by using a depilation needle as shown in Figure 1 or Figure 2, it is possible to deliver an electrical current directly to a region below the skin surrounding a hair follicle without passing current through surface tissue, and thus it will accordingly be appreciated that marking due to current passing through surface tissue is avoided.

In order to further illustrate the invention, reference will now be made to the accompanying Examples.

## EXAMPLE I

A surgical stainless steel depilation needle was coated with a coating of a Xylan 8110 material. The needle was sprayed using a Binks Model 62 spray gun having a No.66 fluid nozzle and a No.66SD air nozzle. The coating was uniformly provided on the needle or probe. In order to ensure that the insulating material was firmly bonded to the needle, curing was effected by heating the needle at 450°F (230°C) for 10 minutes, followed by heating the needle at 600°F (315°C) for 1 minute. The resulting coating of the insulating material was hard and it was satisfactorily adhered to the needle.

The needle was immersed in cold distilled water for twenty four hours. No significant change was found in the pH of the distilled water. The distilled water was then subsequently boiled for twenty minutes. The boiled distilled water was found to retain its original clarity, colour and taste.

This Example thus demonstrates that the insulating material is unlikely to cause irritation to a person's skin or to have any adverse affects on contact with the person's skin.

## EXAMPLE II

A sample of the insulating material referred to in Example I above, was brushed on to a polythene film to give a coated area of approximately 1cm². The coated area was allowed to dry for 48 hours and it was then applied to a person's skin in the bend of the elbow and held in place overnight with an elastoplast strip. No reddening or other sign of irritation was observed on removal of the strip. This demonstrates again the fact that the insulating material is unlikely to cause irritation or to have other adverse effects on a person's skin.

## EXAMPLE III

A needle produced as mentioned above in Example 1 was examined to see if the insulating material could harbour micro-organisms that could infect a person's skin. No evidence was found that the insulating material was unduly susceptible to micro-organisms. Any micro-organisms that might get on to the surface of the insulating material would normally be effectively destroyed by the sterilisation protocol routinely applied with the needles.

It is to be appreciated that various modifications may be made to the needle shown without departing from the scope of the invention and, for example, curved needles may be used. Although references have been made above to a needle, it is to be understood that the device of the invention may alternatively be regarded as a probe if desired. Apart from the coating of the insulating material, the needle may otherwise be of various known designs so that needles of the present invention may be used in a variety of known depilatory machines if desired. Also, the needles may be made of materials other than surgical stainless steel. Other polyamide/polyimide resins, for example Xylan 8114, may be used. The Xylan polyamide/polyimide resins will usually be of such a consistency that dilution will not be necessary. Solvents may however be employed if the insulating material becomes too thick for satisfactory spraying.

## Claims

1. A depilation needle comprising an elongate conductive body the surface of which is covered with insulating material, the body having at one end a conductive tip suitable for depilation purposes and at the other end a conductive surface portion which communicates electrically with the tip via the body, the needle being such that during depilation the conductive tip and the insulating material adjacent the conductive tip are able to contact a person's skin whereby the insulating material prevents the person's skin receiving an electrical depilation current except from the conductive tip, and the insulating material comprising a non-toxic polyamide/polyimide resin.

2. A depilation needle according to claim 1 in which the insulating material includes a lubricant material and/or a pigment.

3. A depilation needle according to claim 2 in which the insulating material includes polytetrafluoroethylene as a lubricant material, and carbon black as a pigment.

4. A depilation needle according to any one of the preceding claims in which the resin is a Xylan resin.

5. A depilation needle according to any one of the preceding claims in which the insulating material is in the form of a lacquer or varnish.

6. A depilation needle according to any one of the preceding claims and which is bent or cranked.

7. A depilation needle according to any one of the preceding claims and which is made of stainless steel.

8. A depilation needle according to any one of the preceding claims in which the tip is one millimetre in length.

9. A depilation needle according to any one of the preceding claims in which the tip comprises a cylindrical extension of the body having a reduced diameter in order to permit insertion between a hair and its pore.

EP 0 380 229 A1

FIG 1

FIG 2

# EUROPEAN SEARCH REPORT

European Patent Office

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| Y,D | GB-A-2 136 296 (LEWIS) <br> * Page 1, lines 35-50 * <br> --- | 1,5-9 | A 61 B 17/41 |
| Y | WO-A-8 403 829 (KÖZPONTI) <br> * Page 6, lines 22-28 * <br> ----- | 1,5-9 | |

|  |  |  | TECHNICAL FIELDS SEARCHED (Int. Cl.5) |
|---|---|---|---|
|  |  |  | A 61 B <br> A 61 H |

The present search report has been drawn up for all claims

| Place of search | Date of completion of the search | Examiner |
|---|---|---|
| THE HAGUE | 09-04-1990 | MOERS R.J. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P0401)